(19) **Europäisches Patentamt** / **European Patent Office** / **Office européen des brevets**

(11) **EP 4 229 223 B1**

(12) # EUROPEAN PATENT SPECIFICATION

(45) Date of publication and mention of the grant of the patent:
**20.03.2024 Bulletin 2024/12**

(21) Application number: **21789765.1**

(22) Date of filing: **14.10.2021**

(51) International Patent Classification (IPC):
**C12Q 1/689** (2018.01)    **G16H 50/30** (2018.01)

(52) Cooperative Patent Classification (CPC):
**C12Q 1/689; G16H 50/30;** Y02A 90/10

(86) International application number:
**PCT/EP2021/078480**

(87) International publication number:
**WO 2022/079184 (21.04.2022 Gazette 2022/16)**

(54) **DEVELOPMENT OF A PERSONALISED PERIODONTITIS SCORE FOR PATIENT RISK STRATIFICATION AND TARGETED THERAPY**

ENTWICKLUNG EINES PERSONALISIERTEN PERIODONTITIS-SCORES ZUR STRATIFIZIERUNG DES RISIKOS EINES PATIENTEN UND GEZIELTE THERAPIE

ÉLABORATION D'UN SCORE DE PARODONTITE PERSONNALISÉ POUR LA STRATIFICATION DES RISQUES D'UN PATIENT ET THÉRAPIE CIBLÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2020 PCT/EP2020/078896**

(43) Date of publication of application:
**23.08.2023 Bulletin 2023/34**

(73) Proprietor: **Parox GmbH**
**04103 Leipzig (DE)**

(72) Inventors:
• **GAGER, Yann**
**04109 Leipzig (DE)**
• **CARVALHO SANTOS, Pablo Sandro**
**Leipzig 04229 (DE)**
• **GABERT, Jörg**
**Leipzig 04155 (DE)**
• **KEBSCHULL, Moritz**
**Bristol Road Birmingham West Midlands B5 7UB (GB)**

(74) Representative: **Maikowski & Ninnemann Patentanwälte Partnerschaft mbB**
**Postfach 15 09 20**
**10671 Berlin (DE)**

(56) References cited:
WO-A1-2019/088272

• **MARCHESAN J.T. ET AL: "Distinct Microbial Signatures between Periodontal Profile Classes", JOURNAL OF DENTAL RESEARCH, vol. 100, no. 12, 27 April 2021 (2021-04-27), pages 1405-1413, XP055877234, US ISSN: 0022-0345, DOI: 10.1177/00220345211009767 Retrieved from the Internet: URL:http://journals.sagepub.com/doi/full-x ml/10.1177/00220345211009767>**
• **VINCENT MEURIC ET AL: "Signature of Microbial Dysbiosis in Periodontitis", APPLIED AND ENVIRONMENTAL MICROBIOLOGY, vol. 83, no. 14, 15 July 2017 (2017-07-15), XP055754715, US ISSN: 0099-2240, DOI: 10.1128/AEM.00462-17**
• **AL-ABDALY MOHAMMED M. A. ABDULLAH ET AL: "The Impact of Age and Gender on Severity and Types of Periodontal Diseases among Patients from Two Regions in Saudi Arabia", OPEN JOURNAL OF STOMATOLOGY, vol. 09, no. 03, 2019, pages 39-53, XP055877047, ISSN: 2160-8709, DOI: 10.4236/ojst.2019.93005 Retrieved from the Internet: URL:https://www.scirp.org/pdf/OJST_2019031 115501221.pdf>**

• CAMELO-CASTILLO ANNY J. ET AL: "Subgingival microbiota in health compared to periodontitis and the influence of smoking", FRONTIERS IN MICROBIOLOGY, vol. 6, 24 February 2015 (2015-02-24), XP055877072, DOI: 10.3389/fmicb.2015.00119 Retrieved from the Internet: URL:https://www.ncbi.nlm.nih.gov/pmc/artic les/PMC4356944/pdf/fmicb-06-00119.pdf>

• SZYMON P SZAFRANSKI ET AL: "High-resolution taxonomic profiling of the subgingival microbiome for biomarker discovery and periodontitis diagnosis", APPLIED AND ENVIRONMENTAL MICROBIOLOGY, vol. 81, no. 3, February 2015 (2015-02), pages 1047-1058, XP055538161, US ISSN: 0099-2240, DOI: 10.1128/AEM.03534-14

• TALITA GOMES BAÊTA LOURENÇO ET AL: "Microbial signature profiles of periodontally healthy and diseased patients", JOURNAL OF CLINICAL PERIODONTOLOGY, vol. 41, no. 11, 22 September 2014 (2014-09-22), pages 1027-1036, XP055486962, DK ISSN: 0303-6979, DOI: 10.1111/jcpe.12302

• SUSAN YOST ET AL: "Functional signatures of oral dysbiosis during periodontitis progression revealed by microbial metatranscriptome analysis", GENOME MEDICINE,, vol. 7, no. 1, 27 April 2015 (2015-04-27), page 27, XP021215658, ISSN: 1756-994X, DOI: 10.1186/S13073-015-0153-3

• MENG SHI ET AL: "The Subgingival Microbiome of Periodontal Pockets With Different Probing Depths in Chronic and Aggressive Periodontitis: A Pilot Study", FRONTIERS IN CELLULAR AND INFECTION MICROBIOLOGY, vol. 8, May 2018 (2018-05), XP055754602, DOI: 10.3389/fcimb.2018.00124

**Description**

**TECHNICAL FIELD**

The present disclosure concerns the development of personalised periodontitis score to assess the individual level of microbial imbalances in periodontal pockets and/or saliva of a specific individual at a defined stage of treatment, relative to a predicted expected score for the average patient with similar systemic and local medical characteristics and demographic profile. The microbial profile obtained from genetic/genomic information is combined with clinical and demographical information in a mixed-effect model to obtain a predicted value and a standard error for a given set of clinical parameters. The comparison between this predicted average score and the individual patient personalised periodontitis score informs about the relative periodontal disease activity in this site and/or patient and thus allows for the stratification of the local site and/or the patient into risk categories and for the recommendation of targeted patient-specific treatment modalities.

**BACKGROUND**

Periodontitis is the most common non-communicable disease in humans, caused by a destructive interaction between a dysbiotic biofilm and a dysregulated host immune-inflammatory response. Periodontitis leads to tooth loss and is independently associated with pre-mature mortality. Oral diseases (mostly periodontitis) are globally responsible for more years lost to disability than any other human disease. Importantly, the detrimental effects - both in the oral cavity and systemically - are especially critical in patients with rapidly progressing variants of the disease.

Periodontitis is *per se* relatively easy and predictable to treat. The real challenge, however, is to detect/predict those patients with rapidly progressing disease before permanent damage occurs, and to thus identify patients that would benefit from a more intensive therapy. This is especially critical in individuals suffering from co-morbidities related to periodontitis and/or risk factors for periodontal disease. There are currently no diagnostic tools available to address these challenges.

Specifically, whilst substantial published evidence (and in part commercial products) exists pointing to single periodontal bacteria, groups/complexes of bacteria, or ecological measures of microbial balance/imbalance (see Table 1 for examples) as markers of disease versus health, and/or as markers of present disease severity, none of these diagnostic measures takes into account co-morbidities, local and systemic risk factors, or demographic information, allowing for stratification of sites or patients in relation to the relative risk. In summary, previously described molecular microbial measures of periodontitis can give information about the disease state, but do not reveal whether - for a given clinical situation - the level of microbial imbalance is similar to the average patient with similar characteristics, or substantially worse or better. Only this information would allow targeting personalised therapeutic approaches to individual patients and/or sites, reducing costly and harmful over-therapy whilst making sure the affected individuals obtain the interventions they require.

Marchesan et al. 2021 (J. Dent. Res. 100(12):1405-1413) relates to assessment of microbial imbalances in periodontitis.

**DESCRIPTION OF THE INVENTION**

The invention is defined by the scope of the appended claims.

The problem of the invention is solved by the method of claim 1, i.e., by a method for the patient- and site-specific assessment of microbial imbalances in periodontitis whilst accounting for critical clinical and demographic parameters, comprising the steps of

- identification and quantification of bacterial species using genetic workflows in samples obtained from periodontal pockets or saliva;
- calculation of a microbial profile based on the quantity of one or several bacterial species associated with periodontitis or a ratio of the quantities of these bacteria;
- using a reference database, modelling of the microbial profile as a function of a selection of relevant clinical and demographical parameters;
- using a set of clinical and demographical parameters from a patient, calculation of the predicted value of the microbial profile via the aforesaid model;
- comparison of the microbial profile between the predicted value and the patient value obtained in the laboratory to assess the relative imbalance of the local microbiome and based on this, recommend a site and/or patient-specific therapy.

The present invention relies on the integrated analysis of several key points to allow for an innovative stratification and personalised targeted therapy of patients suffering from untreated or treated periodontitis, or that could be at risk of developing periodontitis.

The *Personalised Periodontitis Score* is dependent on many parameters, e.g. selected from:

- the genetic/genomic method to measure microbial profiles
- the selection of bacterial species assessed in the microbial profile
- the local and systemic clinical, as well as demographic parameters for the patient or site (e.g., a sample from an 8mm pocket from a molar in supportive periodontal therapy [local clinical factors] from a non-smoking, systemically healthy [systemic clinical factors] white Caucasian male of 51 years [demographic factors]), and
- the data available in the reference database.

Biological samples to be used in the method are samples from periodontal pockets or saliva. All types of periodontal pockets can be sampled. This requires a dental professional. Saliva sampling is very straightforward and does not require substantial infrastructure or skilled personnel. There is no limitation to gender, age and origin, or stage of therapy.

Clinical information means all relevant medical information associated with each sample/patient. Relevant clinical parameters for the present disclosure include:

- Local factors: pocket depth of the sampled site (continuous, in mm), bleeding-on-probing (dichotomous), tooth type (categories: anterior, premolar, molar), therapy stage (categories: untreated, in active therapy, in supportive therapy)
- Systemic factors smoking status (categories: current, past, never) as well as diabetes status (categories: yes/controlled, yes/uncontrolled, no).

Demographic information is information about groups of people. Relevant demographic parameters for the present disclosure include:

- age (continuous, in years),
- gender (categories: male, female, transgender), and
- (optionally) race and ethnicity.

Genetic/genomic workflows comprise all the steps from nucleic acid extraction to the quantification of the sample based on different genetic methods including DNA-DNA hybridisation, quantitative PCR and different Next-Generation Sequencing methods.

A microbial profile is the quantity of one bacterial species or the sum of several bacterial species identified as key markers of periodontitis or periodontal health, or, as an extension, ratios between the sums of selected species. A microbial profile can be calculated based on the number of any bacterial species, which are identified as key markers of periodontitis or periodontal health. Several bacterial species mean a selection of two to 700 bacterial species (i.e. the number of common phylotypes known in the human mouth). The number of bacterial species identified as key markers is at least one, preferably between 2 to 700, more preferably between 2 to 600, 2 to 500, 2 to 400, 2 to 300, 2 to 200 or 2 to 100. Most preferably the number of bacterial species identified as key markers is between 2 to 90, 2 to 80, 2 to 70, 2 to 60, 2 to 50, 2 to 40, 2 to 30, 2 to 20 or 2 to 10. In an especially preferred embodiment, the number of bacterial species identified as key markers is 1, 2, 3 or 4 and the bacterial species are those as listed in Table 1. The unit of the microbial profile is based on the genetic/genomic method used. As a matter of example, the unit of the microbial profile Next-Generation Sequencing (NGS) is NGS reads.

The reference database compiles the following information about multiple samples from different sites and patients:

- Local and systemic clinical information, as outlined above
- Demographical information, as outlined above
- as well as microbial profile for a given genetic/genomic method and selection of bacterial species

This reference database is an essential prerequisite for the calculation of the *Personalised Periodontitis Score.*

A given combination of clinical and demographic information can be used to stratify the sites and/or patients from the reference database. Specific stratifications are expected to have higher values - indicating higher levels of microbial imbalances and thus increased disease activity - of the *Personalised Periodontitis Score* because patients present one or several risk factors like age and smoking status that are well-established risk factors for the disease. A typical stratification for the reference database could be smoking males between 50 and 59 years.

A set of defined clinical and diagnostic parameters are required to calculate the average expected score and the *Personalised Periodontitis Score* for a given genetic/genomic method and selection of microbial profile. As a matter of example in this disclosure, we calculated the scores for non-smoking males of 51 years for three microbial profiles. The linear mixed-effect model for this selection of parameters on the reference database is used to calculate an average expected score as well as associated standard errors to this average. The linear mixed-effect model used in the method of the present invention is known to the person skilled in the art and is described Brown, 2021, the disclosure of which is incorporated herein in its entirety, especially in regard to the description of the linear mixed-effect model on pp. 1 to 19. The Personalised Periodontitis Score from the sampling site is calculated directly with the selected genetic method (quantity of one or several bacterial species) and compared to the average expected score and associated standard errors obtained with the modelling and the reference database.

A *Personalised Periodontitis Score* within -1 SE and 1 SE of the predicted average expected score represents the most typical range of values. In a preferred embodiment, decreased scores have a *Personalised Periodontitis Score* below -1 SE of the predicted value. In a further preferred embodiment, increased scores have a *Personalised Periodontitis Score* below -1 SE of the predicted value.

On the basis of the above, quantities of bacteria can be detected using diverse molecular methods including "checkerboard" DNA-DNA hybridization, quantitative PCR, 16S amplicon sequencing, DNA whole genome sequencing/metagenomics, and/or RNA meta-transcriptomic sequencing. The present invention relies on the detection of microbial profiles which are measures of absolute or relative quantities of one or several marker bacteria associated with periodontitis or periodontal health (Table 1). All types of microbial profiles can be used for the present invention. Sometimes, a *Microbial Dysbiosis Index* or a *Subgingival Microbial Dysbiosis Index* is used as microbial profile in the method of the invention. A suitable *Microbial Dysbiosis Index* is e.g. described in Gevers, Kugathasan et al., 2014, which is incorporated herein in its entirety. A suitable Subgingival Microbial Dysbiosis *Index* is e.g. described in *Chen, Marsh et al.,* 2021, which is incorporated herein in its entirety. The term microbial profile used in the rest of the document refers interchangeably to one of the molecular indices of *Table 1* or any other microbial profile possible.

A *Microbial Dysbiosis Index* relates to the abundance of disease-related microorganism in patient samples and is defined as the log of [total abundance of microorganism increased in periodontitis] over [total abundance of organisms decreased in periodontitis] for all samples. The *Microbial Dysbiosis Index* shows a strong positive correlation with clinical disease severity and negative correlation with species richness, demonstrating that a severe disease state manifests a strongly reduced species diversity in favor of a more extreme dysbiosis.

A *Subgingival Microbial Dysbiosis Index* is defined as the mean centered log-ratio abundance of periodontitis-associated species/genera subtracted by that of health-associated species/genera and can be calculated according to the following method/formula: mean CLR abundance of dysbiotic DS/DG - mean CLR abundance of normobiotic DS/DG. The *Subgingival Microbial Dysbiosis Index* shows a high reproducibility, a strong positive correlation with clinical disease severity and discriminates between periodontitis and health with high accuracy.

*Table 1*: Examples of microbial profiles. A microbial profile is a measure of the quantity of one or several bacterial species identified as key markers of periodontitis or periodontal health, or the ratios of selected species. All of these microbial profiles are in the public domain and this disclosure does not intend to make any claims towards any of these profiles. These measures are merely meant to illustrate the use of the invention, the *Personalised Periodontitis Score* that compares any microbial profile obtained from a specific patient sample using the aforementioned molecular technologies to the expected average score of patients with similar characteristics, aiming to identify those samples that deviate significantly from this average for altered therapy.

| Profile Number | Name | Bacterial species and/or reference |
|---|---|---|
| #1 | - | *Porphyromonas gingivalis* |
| #2 | *Red complex* | *Porphyromonas gingivalis + Tannerella forsythia + Treponema denticola* |
| #3 | *Etiologic bacterial complex* | *Aggregatibacter actinomycetemcomitans + Porphyromonas gingivalis + Tannerella forsythia + Treponema denticola* |
| #4 | *Microbial Dysbiosis Index* | *(Gevers, Kugathasan et al. 2014)* |
| #5 | *Subgingival Microbial Dysbiosis Index* | *(Chen, Marsh et al. 2021)* |

On the basis of the above, the present invention relies on a combination of a microbial profile, such as one of the profiles given in Table 1, with local and systemic clinical, as well as demographic parameters about sites and/or patients. As outlined above, clinical information means all relevant medical information associated with each sample/patient. Relevant clinical parameters for the present disclosure include:

- Local factors: pocket depth of the sampled site (continuous, in mm), bleeding-on-probing (dichotomous), tooth type (categories: anterior, premolar, molar), therapy stage (categories: untreated, in active therapy, in supportive therapy)
- Systemic factors smoking status (categories: current, past, never) as well as diabetes status (categories: yes/controlled, yes/uncontrolled, no).

Demographic information is information about groups of people. Relevant demographic parameters for the present disclosure include:

- age (continuous, in years),
- gender (categories: male, female, transgender), and
- (optionally) race and ethnicity.

A linear mixed-effect model is then used to calculate a *Personalised Periodontitis Score* for the individual microbial profile, taking into account the aforementioned clinical and demographic characteristics and possibly multiple samples per patient. Linear mixed-effect models are an extension of simple linear models to allow both fixed effects (e.g pocket depth which has a defined range) and random effects (e.g., multiple samples per subject). Simple linear models include a simple linear regression where a dependant variable Y can be modelled by a dependant variable X. The response variable in the mixed model is the *Personalised Periodontitis Score* for the individual patient or site. Clinical and demographic parameters represent the explanatory variables in the model. Pocket depth, gender, age, smoking status and diabetes status are fixed effects while patient identity is a random effect. In detail, the equation of the linear mixed-effect model can be expressed as follows:

$$Personalised\ Periodontitis\ Score \sim Local\ clinical\ factors + Systemic\ clinical\ factors +$$

$$Demographical\ factors + (1 \mid patient)$$

The parameters from the equation have the following units:

- Local clinical factors: pocket depth of the sampled site (continuous, in mm), bleeding-on-probing (dichotomous), tooth type (categories: anterior, premolar, molar), therapy stage (categories: untreated, in active therapy, in supportive therapy)

- Systemic clinical factors smoking status (categories: current, past, never) as well as diabetes status (categories: yes/controlled, yes/uncontrolled, no).

- Demographic information is information about groups of people. Relevant demographic parameters for the present disclosure include: age (continuous, in years), gender (categories: male, female, transgender), and (optionally) race and ethnicity.

- Patient - numerical identifier in the clinical database (e.g., insurance number)

On the basis of the above, the site- or patient-related *Personalised Periodontitis Score* obtained in the laboratory from a patient can be compared to an average expected score with the same clinical and demographic parameters of all patient samples previously. As a matter of example, the set of clinical parameters can be defined as follows e.g., pocket depth = 5mm, gender = male, age = 51 years, smoking status = non-smoking and diabetes status = yes. The predictive value of the model based on determined clinical parameter represents a value corrected from many possible confounding factors. The model is also used to calculate +/-1 standard error (SE) around the predicted value as a measure of uncertainty. The predictive value as well as the standard error can be theoretically calculated for all combination of clinical parameters incorporated in the model.

For a given set of clinical parameters, the predicted expected average score and the standard error can be used as a reference to compare to the *Personalised Periodontitis Score* of a patient or site obtained in the laboratory.

The *Personalised Periodontitis Score* is a single value that can be measured repeatedly for the same sampling site/patient to determine and monitor the stage of the disease during all stages of treatment, including in primary and secondary prevention. In other words, the values of the score for different samples taken at the same site but at different times can be compared.

The *Personalised Periodontitis Score* has the advantage that it can be systematically calculated for periodontal pockets or saliva of patients based on different molecular methods. In other words, it is possible to calculate and compare *Personalised Periodontitis Score* for the same molecular method.

While the model is established, the predicted value and the standard error of the *Personalised Periodontitis Score* based on a set of clinical parameters are dynamic. These values vary when adding new entries to the utilised reference database. The predictions gain in certainty with time considering that an increase of data points is associated with a reduction of the variance.

A patient- and site-specific characterization (tooth or oral cavity) is performed by comparing the *Personalised Periodontitis Score* for a specific periodontal pocket or oral cavity (via multiple samples and/or saliva sampling) to the predicted score for a specific set of clinical parameters using the linear mixed-effect model. Patients with samples showing decreased or increased *Personalised Periodontitis Scores* show a microbial imbalance and will receive targeted treatment differing from the treatment schedule recommended for the patients with *Personalised Periodontitis Scores* similar to the average predicted score.

Targeted treatment includes regular treatment and supervision without recommendation of additional anti-microbial measures, intensive treatment/supervision including antiseptic use as well as targeted use of local or, for generalised disease, systemic antibiotics.

Specifically, a sample characterised by a *Personalised Periodontitis Score* increased by more than one standard errors versus the predicted expected average score for a given clinical and demographic situation will be considered to exhibit increased microbial imbalances.

Conversely, for a given set of clinical and demographic parameters, a score of the *Personalised Periodontitis Score* decreased more than 1 SE versus the predicted average expected score represents the part of the population with lower-than-average microbial imbalances, in other words a periodontal pocket showing a profile that is more symbiotic than the rest of the sampled population.

Lastly, for a given set of clinical and demographic parameters, a *Personalised Periodontitis Score* within -1 SE and 1 SE of the predicted average expected score represents the most typical range of values, in other words, a situation showing a profile that is of the population with average microbial balance, in other words a periodontal pocket showing an average profile for dysbiosis in the sampled population.

This approach delivers representative and generalisable insights into the site- and patient-specific levels of microbial balance and allows for targeted treatment recommendations, including personalised intensive approaches including guided localised or systemic adjunctive antiseptics or antibiotics.

A microbial imbalance can be categorized as follows:

*Personalised Periodontitis Score* below -1 SE of the average expected score: Regular treatment and supervision without recommendation of additional anti-microbial measures *Personalised Periodontitis Score* between -1 SE + 1 SE: mild microbial imbalance (corresponds to an early dysbiosis stage), requires intensive treatment/supervision including antiseptic use

*Personalised Periodontitis Score* above +1 SE: severe microbial imbalance (corresponds to advanced dysbiosis stage), requires very intensive treatment/tightly scheduled supervision, including the targeted use of local or, for generalised disease, systemic antibiotics

In addition to absolute measures of the level of symbiosis/dysbiosis, as detailed above, a patient- and site-specific characterization is performed by comparing the *Personalised Periodontitis Score* for a specific periodontal pocket or oral cavity to the average expected score for this clinical severity based on the data in the reference database, whilst taking into account the aforementioned confounders (local and systemic clinical factors, demographical factors) using regressions. Specifically, a sample characterised by one standard errors higher or lower *Personalised Periodontitis Score* than the average expected score for a given disease severity will be considered to exhibit increased or strongly increased dysbiosis or symbiosis, respectively. This approach delivers representative and generalisable insights into the site- and patient-specific dysbiosis levels and allows for targeted treatment recommendations, including personalised intensive approaches including guided localised or systemic adjunctive antiseptics or antibiotics.

The method of invention, in particular the *Personalised Periodontitis Score* has the advantage that it can be used to determine the individual level of microbial imbalance in periodontal pockets and/or the oral cavity and to correctly stratify individuals and/or sites for targeted personalized treatment interventions. These can include various anti-infective and/or periodontal maintenance interventions by the dentists or the dental team to influence microbial imbalances where they exist, and thus to avoid over-treatment, but also to avoid unnecessary and irreversible loss of periodontal tissues.

The method of invention, in particular the *Personalised Periodontitis Score* can, in addition, also be used to estimate the host response, since even though it measures relative quantities of microbial profiles, these were shown to be influenced directly by disproportionate host responses.

The method of invention, in particular the *Personalised Periodontitis Score* has the advantage that it can be used for monitoring the evolution of the stage and the development of the microbial balance for given periodontal pockets or the entire oral cavity over time. A biological sample from one of multiple periodontal pockets or a saliva sample can be sampled for given time intervals to monitor the evolution of the microbial balance in the periodontal pocket or oral cavity. Suitable and preferred time intervals for taking samples are e.g. every 1 day, every 2 days, every 3 days, every 4 days, every 5 days, weekly, 2-weekly or 3-weekly or monthly up to 5 months.

The method of invention, in particular the *Personalised Periodontitis Score* has the advantage that it can be used to check and monitor the effectiveness of treatment to a given patient. After a given treatment, the *Personalised Periodontitis Score* should decrease as a consequence of an improvement of the microbial balance. In the case of similar or higher values of *Personalised Periodontitis Score,* the switch to another adapted antibiotic is a recommended strategy to improve the treatment success. The monitoring of the efficacy of a periodontitis treatment preferably comprises the analysis of samples after or during the treatment that are taken from a periodontal pocket in a predetermined time interval, such as every 1 day, every 2 days, every 3 days, every 4 days, every 5 days, weekly, 2-weekly or 3-weekly or monthly up to 5 months.

The method of invention, in particular the *Personalised Periodontitis Score* has the advantage that it can be used to compare results from different sources for example dental practices or research teams. The complexity of microbial communities in periodontal pockets is summarized into a single number (predicted value) and range (-1 SE to +1 SE) for a given method that is self-explanatory and does not reference to other samples or databases. The method of invention, in particular the *Personalised Periodontitis Score* has the advantage that it can be used as a proxy to monitor other diseases. Indeed, periodontitis has been associated to a vast number of diseases including diabetes mellitus, cardiovascular diseases like coronary heart disease and stroke, chronic respiratory diseases and rheumatoid arthritis. Patients showing high values of the *Personalised Periodontitis Score* characterizing relative microbial imbalances in periodontal pockets or the oral cavity may present higher risks to all the diseases mentioned above.

The molecular methods for detecting bacteria in periodontal pockets are known. These methods consist of "checkerboard" DNA-DNA hybridisation, quantitative PCR and Next-Generation Sequencing including 16 S ribosomal RNA sequencing, metagenomics and metatranscriptomics, among others.

Further disclosed but not part of the invention is a combination of microbial profiles, comprised of one or more bacterial species which are key markers of periodontitis and/or periodontal health.

Disclosed but not part of the invention are all other microbial profiles resulting from one or several key bacteria associated positively or negatively with periodontitis, including ecological measures of microbial imbalances, such as indices quantifying microbial dysbiosis.

The invention is now further illustrated in three working examples illustrated by three figures for the microbial profile which comprises of 1) *Porphyromonas gingivalis,* 2) the Red complex (*Porphyromonas gingivalis* + *Tannerella forsythia* + *Treponema denticola*) and 3) the Etiologic bacterial complex (*Aggregatibacter actinomycetemcomitans* + *Porphyromonas gingivalis* + *Tannerella forsythia* + *Treponema denticola*).

## SHORT DESCRIPTION OF THE FIGURES

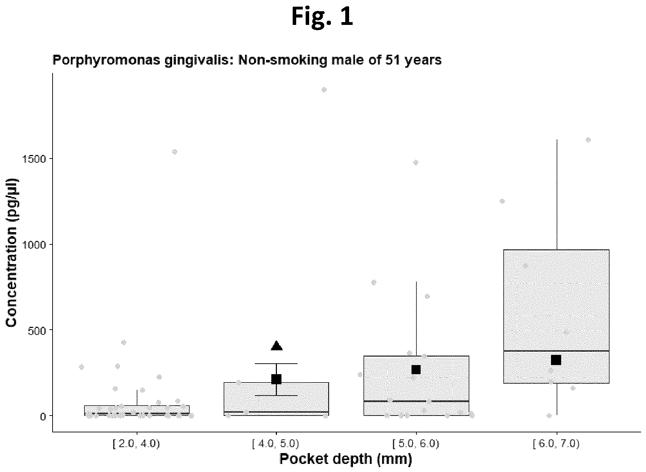

**Figure 1**    shows for *Porphyromonas gingivalis* the predicted values for a non-smoking male of 51 years (represented as squares) based on the model mentioned above and calculations based on a quantitative PCR dataset associated with clinical data. As a matter of example, the standard error around the predicted values and a fictive patient value (represented as triangle) is represented for the pocket depth 4 mm. The fictive patient value being above + 1 SE of the average expected score, the patient requires very intensive treatment/tightly scheduled supervision.

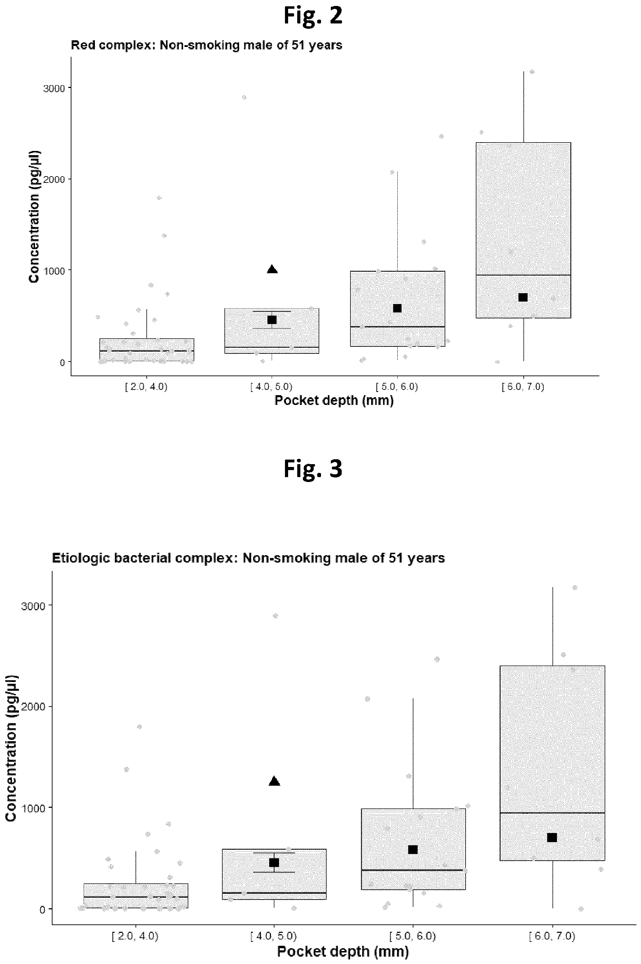

**Figure 2**    shows for the Red complex the predicted values for a non-smoking male of 51 years (represented as squares) based on the model mentioned above and calculations based on a quantitative PCR dataset associated with clinical data. As a matter of example, the standard error around the predicted values and a fictive patient value (represented as triangle) is represented for the pocket depth 4 mm. The fictive patient value being above + 1 SE of the average expected score, the patient requires very intensive treatment/tightly scheduled supervision.

**Figure 3**    shows for the Etiologic bacterial complex the predicted values for a non-smoking male of 51 years (represented as squares) based on the model mentioned above and calculations based on a quantitative PCR dataset associated with clinical data. As a matter of example, the standard error around the predicted values and a fictive patient value (represented as triangle) is represented for the pocket depth 4 mm. The fictive patient value being above + 1 SE of the average expected score, the patient requires very intensive treatment/tightly scheduled supervision.

**EXAMPLE 1: PREDICTIVE MODELLING OF A MOLECULAR**

*Personalised Periodontitis Score* **ON QUANTITATIVE PCR DATA**

Materials and methods for the calculation of the microbial profile and modelling

The dataset comes from the supplementary material "DATA SHEET S1 | Dataframe and univariate analysis." from the scientific paper:
Tomás I, Regueira-Iglesias A, López M, Arias-Bujanda N, Novoa L, Balsa-Castro C and Tomás M (2017) Quantification by qPCR of Pathobionts in Chronic Periodontitis: Development of Predictive Models of Disease Severity at Site-Specific Level. Front. Microbiol. 8:1443. doi: 10.3389/fmicb.2017.01443
The dataset consists of quantitative PCR (qPCR) information of different bacteria associated with periodontitis (columns "Concentration pg" in tab "qPCR data"). The dataset also contains clinical information (tab "Clinical Data") including patient identity, age, gender, smoking status (in the four first columns) as well as pocket depth (both for control site in column "PPD_Control" and periodontitis site in column "PPD_Perio"). Information about the diabetes status was not available in the dataset and could therefore not be included in the analysis.

*Statistics*

All analyses were done using the software R with custom-designed scripts. The clinical and qPCR information were matched thanks to the Patient ID. The following microbial profiles from Table 1 were calculated to illustrate the invention:

- Profile #1 was the concentration of the bacterium *Porphyromonas gingivalis* in pg
- Profile #2 - *Red Complex* was calculated as the sum of the columns "Concentration pg" for the bacteria *Porphyromonas gingivalis, Tannerella forsythia* and *Treponema denticola.*
- Profile #3 - *Etiologic bacterial burden* was calculated as the sum of the columns "Concentration pg" for the bacteria *Aggregatibacter actinomycetemcomitans + Porphyromonas gingivalis + Tannerella forsythia + Treponema denticola*

The package *lme4* from R was used to create the following linear mixed-effect model to obtain the average expected as well as associated standard errors to this average *Personalised Periodontitis Score* for a given category of parameters:

lmer(Microbial profile ~ Pocket depth + Gender + Age + Smoking status + (1 | patient ID))

The model was then used to obtain a mean predicted value as well as the standard error for the different molecular profiles used for this illustration for a defined set of parameters: pocket depth between 4 and 10 mm, male as gender, 51 years as age and non-smoking as smoking status. The choice of the values from 4 to 10 mm pocket depth represents mild, moderate and severe cases, without those at risk to stem from periodontal-endodontic lesions with a different pathophysiology. The predicted value of the microbial profile for this set of clinical parameters was positively correlated with pocket depth for all used microbial indices, as expected.
The graphics can then be used to compare values for the three different microbial profiles. In this case we take the example of 400 pg/$\mu$l for profile #1, 1000 pg/$\mu$l for profile #2 and 1250 pg/$\mu$l for profile # for a pocket depth of 4 mm and the other clinical parameters comparable. For the three microbial profiles, the value is definitely above +1 SE of the average expected score. In these situations, the pocket would have a very dysbiotic profile which would require a very intensive treatment possibly with antibiotics as well as tightly scheduled supervision.

**References**

Brown, V. A. (2021). "An Introduction to Linear Mixed-Effects Modeling in R." Advances in Methods and Practices in Psychological Science 4(1): 1-19.

Chen, T., P. D. Marsh and N. N. Al-Hebshi (2021). "SMDI: An Index for Measuring Subgingival Microbial Dysbiosis." J Dent Res: 220345211035775.

Gevers, D., S. Kugathasan, L. A. Denson, Y. Vázquez-Baeza, W. Van Treuren, B. Ren, E. Schwager, D. Knights, S. J. Song, M. Yassour, X. C. Morgan, A. D. Kostic, C. Luo, A. González, D. McDonald, Y. Haberman, T. Walters, S. Baker, J. Rosh, M. Stephens, M. Heyman, J. Markowitz, R. Baldassano, A. Griffiths, F. Sylvester, D. Mack, S. Kim, W. Crandall, J. Hyams, C. Huttenhower, R. Knight and R. J. Xavier (2014). "The treatment-naive microbiome in new-onset Crohn's disease." Cell Host Microbe 15(3): 382-392.

Tomás I, Regueira-Iglesias A, López M, Arias-Bujanda N, Novoa L, Balsa-Castro C and Tomás M (2017) Quantification by qPCR of Pathobionts in Chronic Periodontitis: Development of Predictive Models of Disease Severity at Site-Specific Level. Front. Microbiol. 8:1443. doi: 10.3389/fmicb.2017.01443

## Claims

1. A method for the patient- and site-specific assessment of microbial imbalances in periodontitis building on clinical and demographic parameters, comprising the steps of

   • identification and quantification of bacterial species using genetic workflows in samples obtained from periodontal pockets or saliva;
   • calculation of a microbial profile based on the quantity of one or several bacterial species associated with periodontitis or a ratio of the quantities of these bacteria;
   • using a reference database, modelling of the microbial profile as a function of a selection of relevant clinical and demographical parameters;
   • using a set of clinical and demographical parameters from a patient, calculation of the predicted value of the microbial profile via the aforesaid model;
   • comparison of the microbial profile between the predicted value and the patient value obtained in the laboratory to assess the relative imbalance of the local microbiome and based on this, recommend a site and/or patient-specific therapy,
   wherein said clinical information includes

   • local factors: pocket depth of the sampled site, bleeding-on-probing, tooth type, therapy stage selected from untreated, in active therapy and in supportive therapy,
   • systemic factors smoking status as well as diabetes status,

   said demographic information includes

   • age,
   • gender, and
   • optionally race and ethnicity,

   and wherein for modelling of the microbial profile, a linear mixed-effect model is used, which is based on molecular and clinical data from a reference database, wherein the model includes the microbial profile as the response variable and clinical parameters including pocket depth as explanatory variables.

2. The method according to claim 1, wherein the microbial profile is defined as the quantity of one or several bacterial species identified as key markers of periodontitis, wherein several bacterial species mean a selection of two to 700 bacterial species, which represent the number of common phylotypes known in the human mouth.

3. The method according to claim 1, wherein the microbial profile is selected from ecological measures of microbial imbalances, such as indices quantifying microbial dysbiosis, specifically a *Microbial Dysbiosis Index* or a *Subgingival Microbial Dysbiosis Index.*

4. The method according to any one of claims 1 to 3, wherein said linear mixed-effect model is used to predict the microbial profile as well as the standard error for any combination of parameters used in the model.

5. The method according to any one of claims 1 to 4, wherein the microbial profile of an additional patient with defined clinical parameters can be compared to the predicted value and the standard error based on the exact same of clinical parameters

6. The method according to any one of claims 1 to 5, wherein a microbial imbalance stage is defined as follows:

*Personalised Periodontitis Score* < -1 SE from predicted value healthy stage
*Personalised Periodontitis Score* between -1/+1 SE from predicted value early dysbiosis stage
*Personalised Periodontitis Score* > + 1 SE from predicted value advanced dysbiosis stage

7. The method according to any one of the preceding claims, wherein the bacterial species are identified by quantitative PCR, 16 S ribosomal RNA sequencing, shotgun sequencing or any other molecular techniques.

8. Use of the method according to any one of the preceding claims for determining the severity of periodontitis.

9. Use of the method according to claims 1 to 7 for monitoring dysbiosis development in periodontitis over time.

10. Use of the method according to claims 1 to 7 for monitoring the efficacy of periodontitis treatment.

11. Use of the method according to claims 1 to 7 for recommending and selecting a strategy for the treatment of periodontitis.

12. A method for monitoring dysbiosis development in periodontitis comprising the method as claimed in any one of claims 1 to 7, wherein samples are analysed that are taken from a periodontal pocket in a predetermined time interval, such as every 1 day, every 2 days, every 3 days, every 4 days, every 5 days, weekly, 2-weekly or 3-weekly or monthly up to 5 months.

13. A method for monitoring the efficacy of a periodontitis treatment comprising the method as claimed in any one of claims 1 to 7, wherein samples are analysed after or during the treatment that are taken from a periodontal pocket in a predetermined time interval, such as every 1 day, every 2 days, every 3 days, every 4 days, every 5 days, weekly, 2-weekly or 3-weekly or monthly up to 5 months.

**Patentansprüche**

1. Verfahren für die Patienten- und Stellen-spezifische Bewertung mikrobieller Ungleichgewichte bei Periodontitis (Parodontitis) auf Grundlage klinischer und demografischer Parameter, umfassend die folgenden Schritte:

• Identifizierung und Quantifizierung von Bakterienspezies in aus Parodontaltaschen oder Speichel gewonnenen Proben mithilfe gentechnologischer Verfahrensweisen;
• Berechnung eines mikrobiellen Profils basierend auf der Menge einer oder mehrerer mit Parodontitis assoziierter Bakterienspezies oder einem Verhältnis der Mengen dieser Bakterien;
• Modellieren des mikrobiellen Profils als Funktion einer Auswahl relevanter klinischer und demografischer Parameter unter Verwendung einer Referenzdatenbank;
• Berechnung des vorhergesagten Werts des mikrobiellen Profils anhand des oben erwähnten Modells unter Verwendung einer Reihe klinischer und demografischer Parameter eines Patienten;
• Vergleich des mikrobiellen Profils zwischen dem vorhergesagten Wert und dem im Labor ermittelten Patientenwert, um das relative Ungleichgewicht des lokalen Mikrobioms zu bewerten und darauf basierend eine Stellen- und/oder Patientenspezifische Therapie zu empfehlen,
wobei die erwähnten klinischen Informationen

• lokale Faktoren: Taschentiefe der beprobten Stelle, Blutung beim Sondieren, Zahntyp, Therapiestadium, ausgewählt aus unbehandelt, in-aktiver-Therapie und in-Begleittherapie,
• systemische Faktoren, Raucher-Status sowie Diabetes-Status, umfassen,

wobei die erwähnten demografischen Informationen

• das Alter,
• das Geschlecht und
• optional die Rasse und Ethnizität umfassen,

und wobei zum Modellieren des mikrobiellen Profils ein lineares gemischte-Effekte-Modell angewandt wird, das auf molekularen und klinischen Daten aus einer Referenzdatenbank basiert, wobei das Modell das mikrobielle Profil als Antwortvariable und klinische Parameter, einschließlich der Taschentiefe, als erklärende Variablen umfasst.

2. Verfahren nach Anspruch 1, wobei das mikrobielle Profil als die Menge einer oder mehrerer Bakterienspezies definiert ist, die als Schlüsselmarker für Parodontitis identifiziert wurden, wobei mehrere Bakterienspezies eine Auswahl von zwei bis 700 Bakterienspezies bedeuten, die die Anzahl der im menschlichen Mund bekannten üblichen Phylotypen repräsentieren.

3. Verfahren nach Anspruch 1, wobei das mikrobielle Profil aus ökologischen Messwerten von mikrobiellen Ungleichgewichten ausgewählt ist, wie etwa Indizes zum Quantifizieren der mikrobiellen Dysbiose, insbesondere einem *Microbial Dysbiosis Index* oder einem *Subgingival Microbial Dysbiosis Index*.

4. Verfahren nach einem der Ansprüche 1 bis 3, wobei das erwähnte lineare gemischte-Effekte-Modell zum Vorhersagen des mikrobiellen Profils sowie des Standardfehlers für jedwede im Modell verwendete Kombination von Parametern eingesetzt wird.

5. Verfahren nach einem der Ansprüche 1 bis 4, wobei das mikrobielle Profil eines weiteren Patienten mit definierten klinischen Parametern mit dem vorhergesagten Wert und dem Standardfehler, basierend auf genau den gleichen der klinischen Parameter, verglichen werden kann.

6. Verfahren nach einem der Ansprüche 1 bis 5, wobei ein mikrobielles Ungleichgewicht-Stadium wie folgt definiert ist:

*Personalisierter Periodontitis-Score* < - 1 SE vom vorhergesagten Wert Gesundes Stadium
*Personalisierter Periodontitis-Score* -1/+1 SE vom vorhergesagten Wert Frühes Dysbiose-Stadium
*Personalisierter Periodontitis-Score* > + 1 SE vom vorhergesagten Wert Fortgeschrittenes Dysbiose-Stadium.

7. Verfahren nach einem der vorangehenden Ansprüche, wobei die Bakterienspezies durch quantitative PCR, 16S-ribosomale-RNA-Sequenzierung, Shotgun-Sequenzierung oder beliebige andere molekulare Techniken identifiziert werden.

8. Verwendung des Verfahrens nach einem der vorangehenden Ansprüche zum Bestimmen des Schweregrads der Parodontitis.

9. Verwendung des Verfahrens nach den Ansprüchen 1 bis 7 zur Überwachung der Dysbiose-Entwicklung bei Parodontitis im Zeitverlauf.

10. Verwendung des Verfahrens nach den Ansprüchen 1 bis 7 zur Überwachung der Wirksamkeit einer Parodontitis-Behandlung.

11. Verwendung des Verfahrens nach den Ansprüchen 1 bis 7 zum Empfehlen und Auswählen einer Strategie für die Behandlung von Parodontitis.

12. Verfahren zur Überwachung der Dysbiose-Entwicklung bei Parodontitis, umfassend das Verfahren nach einem der Ansprüche 1 bis 7, wobei Proben analysiert werden, die aus einer Parodontaltasche in einem vorgegebenen Zeitintervall, beispielsweise jeden Tag, alle 2 Tage, alle 3 Tage, alle 4 Tage, alle 5 Tage, wöchentlich, 2-wöchentlich oder 3-wöchentlich oder monatlich während bis zu 5 Monaten, entnommen werden.

13. Verfahren zur Überwachung der Wirksamkeit einer Parodontitis-Behandlung, umfassend das Verfahren nach einem der Ansprüche 1 bis 7, wobei Proben nach oder während der Behandlung analysiert werden, die aus einer Parodontaltasche in einem vorgegebenen Zeitintervall, beispielsweise jeden Tag, alle 2 Tage, alle 3 Tage, alle 4 Tage, alle 5 Tage, wöchentlich, 2-wöchentlich oder 3-wöchentlich oder monatlich während bis zu 5 Monaten, entnommen werden.

**Revendications**

1. Procédé d'évaluation spécifique au patient et à un site de déséquilibres microbiens dans le développement de la parodontite en s'appuyant sur des paramètres cliniques et démographiques, comprenant les étapes de

   • identification et quantification d'espèces bactériennes à l'aide de flux de travail génétiques dans des échantillons obtenus à partir de poches parodontales ou de salive ;
   • calcul d'un profil microbien basé sur la quantité d'une ou plusieurs espèces bactériennes associées à la parodontite ou sur un rapport des quantités de ces bactéries ;
   • utilisation d'une base de données de référence, modélisation du profil microbien en fonction d'une sélection de paramètres cliniques et démographiques pertinents ;
   • à l'aide d'un ensemble de paramètres cliniques et démographiques provenant d'un patient, calcul de la valeur prédite du profil microbien via le modèle susmentionné ;
   • comparaison du profil microbien entre la valeur prédite et la valeur du patient obtenue dans le laboratoire pour évaluer le déséquilibre relatif du microbiome local et, sur cette base, recommander un traitement spécifique au site et/ou au patient, dans lequel lesdites informations cliniques comprennent
   • des facteurs locaux : profondeur de poche du site échantillonné, saignement au sondage, type de dent, stade de traitement sélectionné parmi non traité, en traitement actif et en traitement de soutien,
   • des facteurs systémiques du statut du tabagisme et du statut du diabète,
   lesdites informations démographiques comprennent

      • l'âge,
      • le sexe, et
      • facultativement, la race et l'origine ethnique,

   et dans lequel, pour la modélisation du profil microbien, un modèle linéaire à effet mixte est utilisé, qui est basé sur des données moléculaires et cliniques d'une base de données de référence, dans lequel le modèle inclut le profil microbien comme la variable de réponse et les paramètres cliniques, y compris la profondeur de poche comme variables explicatives.

2. Procédé selon la revendication 1, dans lequel le profil microbien est défini comme la quantité d'une ou plusieurs espèces bactériennes identifiées comme marqueurs clés de la parodontite, dans lequel plusieurs espèces bactériennes signifient une sélection de deux à 700 espèces bactériennes, qui représentent le nombre de phylotypes courants connus dans la bouche humaine.

3. Procédé selon la revendication 1, dans lequel le profil microbien est sélectionné parmi des mesures écologiques de déséquilibres microbiens, tels que des indices quantifiant la dysbiose microbienne, en particulier un *indice de dysbiose microbienne* ou un *indice de dysbiose microbienne sous-gingivale*.

4. Procédé selon l'une quelconque des revendications 1 à 3, dans lequel ledit modèle linéaire à effet mixte est utilisé pour prédire le profil microbien ainsi que l'erreur type pour toute combinaison de paramètres utilisée dans le modèle.

5. Procédé selon l'une quelconque des revendications 1 à 4, dans lequel le profil microbien d'un patient supplémentaire comportant des paramètres cliniques définis peut être comparé à la valeur prédite et à l'erreur type sur la base d'exactement les mêmes paramètres cliniques.

6. Procédé selon l'une quelconque des revendications 1 à 5, dans lequel un stade de déséquilibre microbien est défini comme suit :

   *Score de parodontite personnalisé* < - 1 SE par rapport au stade de santé de la valeur prédite
   *Score de parodontite personnalisé* compris entre -1/+1 SE par rapport au stade de dysbiose précoce de la valeur prédite *Score de parodontite personnalisé* > + 1 SE par rapport au stade de dysbiose avancée de la valeur prédite.

7. Procédé selon l'une des revendications précédentes, dans lequel les espèces bactériennes sont identifiées par PCR quantitative, séquençage de l'ARN ribosomal 16 S, séquençage aléatoire ou de quelconques autres techniques moléculaires.

8. Utilisation du procédé selon l'une quelconque des revendications précédentes pour déterminer la gravité de la parodontite.

9. Utilisation du procédé selon les revendications 1 à 7 pour la surveillance du développement de la dysbiose dans la parodontite au fil du temps.

10. Utilisation du procédé selon les revendications 1 à 7 pour la surveillance de l'efficacité du traitement de la parodontite.

11. Utilisation du procédé selon les revendications 1 à 7 pour la recommandation et la sélection d'une stratégie pour le traitement de la parodontite.

12. Procédé de surveillance du développement de la dysbiose dans la parodontite comprenant le procédé selon l'une quelconque des revendications 1 à 7, dans lequel des échantillons sont analysés provenant d'une poche parodontale dans un intervalle de temps prédéterminé, par exemple tous les jours, tous les 2 jours, tous les 3 jours, tous les 4 jours, tous les 5 jours, de manière hebdomadaire, de manière bi-hebdomadaire ou de manière tri-hebdomadaire ou mensuellement jusqu'à 5 mois.

13. Procédé de surveillance de l'efficacité d'un traitement de parodontite comprenant le procédé selon l'une quelconque des revendications 1 à 7, dans lequel des échantillons sont analysés après ou pendant le traitement qui sont prélevés à partir d'une poche parodontale dans un intervalle de temps prédéterminé, par exemple tous les jours, tous les 2 jours, tous les 3 jours, tous les 4 jours, tous les 5 jours, de manière hebdomadaire, de manière bi-hebdomadaire ou de manière tri-hebdomadaire ou mensuellement jusqu'à 5 mois.

# Fig. 1

Porphyromonas gingivalis: Non-smoking male of 51 years

# Fig. 2

Red complex: Non-smoking male of 51 years

# Fig. 3

Etiologic bacterial complex: Non-smoking male of 51 years

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARCHESAN et al.** *J. Dent. Res.,* 2021, vol. 100 (12), 1405-1413
- **GEVERS, KUGATHASAN et al.** *Subgingival Microbial Dysbiosis,* 2014
- **TOMÁS I ; REGUEIRA-IGLESIAS A ; LÓPEZ M ; ARIAS-BUJANDA N ; NOVOA L ; BALSA-CASTRO C ; TOMÁS M.** Quantification by qPCR of Pathobionts in Chronic Periodontitis: Development of Predictive Models of Disease Severity at Site-Specific Level. *Front. Microbiol.,* 2017, vol. 8, 1443

- **BROWN, V. A.** An Introduction to Linear Mixed-Effects Modeling in R. *Advances in Methods and Practices in Psychological Science,* 2021, vol. 4 (1), 1-19
- **CHEN, T. ; P. D. MARSH ; N. N. AL-HEBSHI.** SMDI: An Index for Measuring Subgingival Microbial Dysbiosis. *J Dent Res,* 2021
- **GEVERS, D ; S. KUGATHASAN ; L. A. DENSON ; Y. VÁZQUEZ-BAEZA ; W. VAN TREUREN ; B. REN ; E. SCHWAGER ; D. KNIGHTS ; S. J. SONG ; M. YASSOUR.** The treatment-naive microbiome in new-onset Crohn's disease. *Cell Host Microbe,* 2014, vol. 15 (3), 382-392